(19) Europäisches Patentamt
European Patent Office
Office européen des brevets

(11) Publication number: **0 264 467 B1**

# (12) EUROPEAN PATENT SPECIFICATION

(45) Date of publication of patent specification: **24.06.92**

(51) Int. Cl.5: **C07D 251/16**, A01N 47/36

(21) Application number: **86114470.7**

(22) Date of filing: **18.10.86**

The file contains technical information submitted after the application was filed and not included in this specification

## (54) Sulfamoyl urea derivates.

(43) Date of publication of application:
**27.04.88 Bulletin 88/17**

(45) Publication of the grant of the patent:
**24.06.92 Bulletin 92/26**

(84) Designated Contracting States:
**AT BE CH DE FR GB GR IT LI NL SE**

(56) References cited:
**GB-A- 2 110 689**
**GB-A- 2 113 217**
**US-A- 4 592 776**

**CHEMICAL ABSTRACTS, vol.99, 1983, Columbus, OH (US); p.563, no.53793m**

(73) Proprietor: **PPG INDUSTRIES, INC.**
**One PPG Place**
**Pittsburgh Pennsylvania 15272(US)**

(72) Inventor: **Van Gemert, Barry**
**7331 Shadyview Avenue**
**Massillon, OH 44646(US)**

(74) Representative: **Sternagel, Hans-Günther, Dr. et al**
**Patentanwälte Dr. Michael Hann Dr. H.-G. Sternagel Sander Aue 30**
**W-5060 Bergisch Gladbach 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 264 467 B1

## Description

The present invention relates to sulfamoyl urea derivatives and to herbicidal formulations containing them. It further relates to a method of controlling the growth of weeds using the above mentioned urea derivatives.

US-A-4,592,776 refers to phenyl sulfamoyl urea derivatives, which compounds are, however, substituted by other functional groups in the phenyl moiety than the compound as claimed. These compounds can be used to control the growth of noxious plants, i.e. weeds.

Chemical Abstracts, vol. 99 (1983) abstract no. 53 793m refers to phenyl sulfonylureide derivatives which compounds are, however, also substituted by other functional groups than those presently claimed. It is further stated that these compounds can be used as selective herbicides and fungicides.

GB-A-2,110,689 relates to heterocyclic aminosulfamyl ureas i.a. anthranylic acid, N-{[(X,Y-s-triazin-2-yl)-carbamoyl]sulfamoyl, lower alkyl esters and anthranylic acid, N-{[(X,Y-2-pyrimidinyl)carbamoyl]sulfamoyl lower alkyl ester, a process for producing these compounds, which compounds can be used as selective herbicides.

GB-A-2,113,217 discloses triaza compounds i.a. anthranylic acid, N-{[(X,Y-2-pyrimidinyl)carbamoyl]-sulfamoyl lower alkyl esters and a process for preparing these compounds, which compounds can be used as selective herbicides.

This invention concerns sulfamoyl urea derivatives represented by the formula:

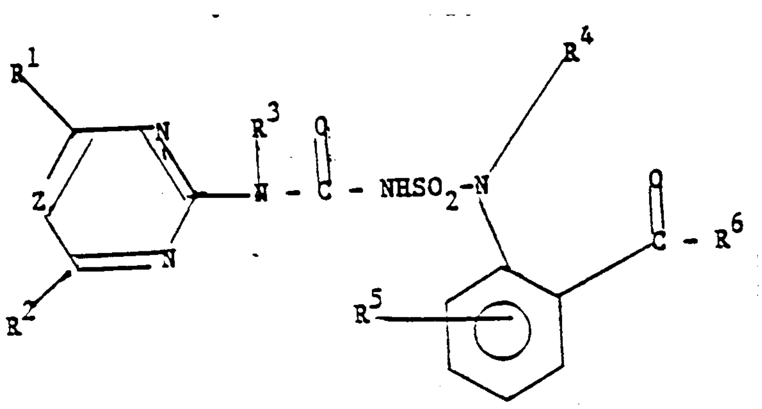

I.

wherein:

| | |
|---|---|
| Z | is N or CH; |
| $R^1$ and $R^2$ | are the same or different and represent halogen or $C_1$ to $C_6$ alkyl or alkoxy; |
| $R^3$ and $R^4$ | are the same or different and represent hydrogen, $C_1$ to $C_4$ alkyl, alkoxyalkyl, haloalkyl, or up to $C_3$ alkenyl or alkynyl; |
| $R^5$ | is hydrogen, halogen or $C_1$ to $C_4$ alkyl, haloalkyl; and |
| $R^6$ | is methyl or ethyl. |

Further provided is a herbicidal formulation containing an agronomically acceptable carrier and a compound or mixture of compounds defined above.

Furthermore, provided is a method of controlling weeds wherein a herbicidally effective amount of herbicide is applied to a growth medium prior to emergence of weeds therefrom or to the weeds subsequent to their emergence from the growth medium,

**characterized by**

using as the herbicide a compound or mixture of compounds defined above.

Preferred compounds within the scope of this invention are those wherein Z is nitrogen, $R^1$ is lower alkyl, e.g., methyl; $R^2$ is lower alkoxy, e.g., methoxy; $R^3$, $R^4$ and $R^5$ are hydrogen; and $R^6$ is methyl.

The Formula I compounds may conveniently be prepared in a two-step reaction, involving reacting in the first step a suitably substituted amino pyrimidine or amino triazine of the Formula II:

II.

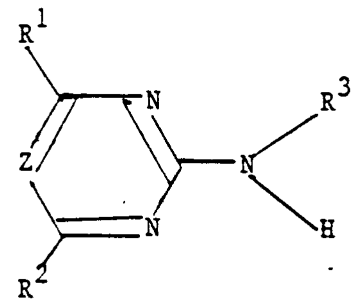

wherein $R^1$, $R^2$, $R^3$ and Z are as previously defined, with a halosulfonyl isocyanate of the formula OCN-$SO_2$-Hal, wherein Hal is halogen, e.g., chlorine, fluorine or bromine to form the corresponding halosulfonyl urea of the Formula III:

III.

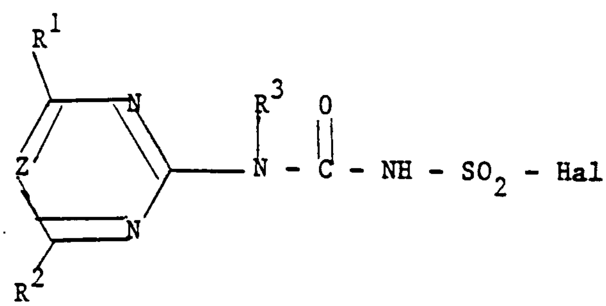

In the second step, the Formula III compound is reacted, preferably in the presence of an acid acceptor, e.g. triethylamine, with at least a stoichiometric amount of a suitably substituted amine of the Formula IV:

IV.

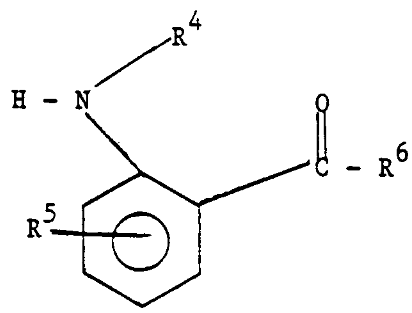

wherein $R^4$, $R^5$ and $R^6$ are as previously defined, to form a Formula I compound of the invention.

The invention is further illustrated by the following Examples which describe preparation of certain compounds of this invention.

The following Examples are illustrative of the preparation of certain preferred compounds of this invention.

Example I

Preparation of: 1-[(o-acetylphenyl)-sulfamoyl]-3-(4-methyl-6-methoxy-1,3,5-triazin-2-yl)urea

To a stirred solution of 0.01 mole of chlorosulfonyl isocyanate in methylene chloride maintained at 0-5 ° C. via an ice bath was added 1.4 grams (0.01 mole) of 2-amino-4-methyl-6-methoxy-1,3,5-triazine. After two hours reaction time, a methylene chloride solution containing 1.35 grams (0.01 mole) of o-amino

acetophenone and 1.0 gram (0.01 mole) of triethylamine is added dropwise. The reaction mixture was stirred rapidly while in the ice bath and was then removed and allowed to warm to room temperature. After standing overnight, the reaction mixture was poured into aqueous sodium carbonate. The aqueous layer was washed once with chloroform and filtered. Acidification with dilute aqueous hydrochloric acid resulted in a white precipitate which after suction filtration and vacuum drying afforded 2.4 grams of material confirmed by NMR analysis as the desired product.

Example II

Preparation of: 1-(2-methylcarbonyl-4-fluorophenylsulfamoyl)-3-(4-methyl-6-methoxy-1,3,5-triazin-2-yl)urea

To 75 milliliters of methylene chloride maintained at 0-5 °C via an ice bath were added 1.4 grams of 4-methyl-6-methoxy-2-amino-1,3,5-triazine and 1.55 grams of chlorosulfonyl isocyanate. After stirring for 2 hours at ice bath temperature a methylene chloride solution containing 1.0 gram of triethylamine and 1.53 grams of 5-fluoro-2-amino-acetophenone was added dropwise. The stirred reaction mixture was permitted to warm to room temperature and was then shaken once with 2 percent aqueous hydrochloric acid then twice with water. Removal of solvent afforded a crystalline solid which was washed with a small amount of diethyl ether. Suction drying afforded light tan crystals identified by NMR analysis as the desired product.

Example III

Preparation of: 1-(2-methylcarbonyl)-4-chlorophenylsulfamoyl)-3-(4-methyl-6-methoxy-1,3,5-triazin-2-yl)urea

To 75 milliliters of methylene chloride maintained at 0-5 °C. via an ice bath were added 1.4 grams of 4-methyl-6-methoxy-2-amino-1,3,5-triazine and 1.55 grams of chlorosulfonyl isocyanate. After stirring for 2 hours at ice bath temperature a methylene chloride solution containing 1.0 gram of triethylamine and 1.7 grams of 5-chloro-2-aminoacetophenone was added dropwise. The stirred reaction mixture was permitted to warm to room temperature and was then shaken with cold, dilute aqueous hydrochloric acid solution. After phase separation, the organic layer was drawn-off, washed with water and evaporated affording a brown pasty residue. The residue was dissolved in 5 milliliters of methylene chloride. Diethyl ether was added and precipitation was initiated by scratching the flask. The crystalline precipitate was isolated by filtration, washed with diethyl ether and suction dried, affording 2.8 grams of white crystals confirmed by NMR analysis to be the desired product.

Although the invention has been illustrated by the foregoing Examples with regard to the preparation of certain compounds within the scope of Formula I, it is to be understood that other compounds within the scope of Formula I may readily be prepared by those skilled in the art simply by varying the choice of starting materials and using the same or similiar techniques.

Weed control in accordance with this invention is effected by applying to the soil prior to emergence of weeds therefrom or to the plant surfaces subsequent to emergence from the soil, a herbicidally effective amount of a compound of this invention. It is, of course, to be understood that the term "a compound of this invention" also includes mixtures of such compounds or a formulation containing a compound or mixture of compounds of this invention.

The term "herbicidally effective amount" is that amount of a compound of this invention required to so injure or damage weeds such that the weeds are incapable of recovering following application while not causing substantial injury to any valuable crop amongst which the weeds might be growing. The quantity of a compound of this invention applied in order to exhibit a satisfactory herbicidal effect may vary over a wide range and depends on a variety of factors, such as, for example, hardiness of a particular weed species, extent of weed infestation, climatic conditions, soil conditions, method of application, and the like. Typically, as little as 1.12 or less Kg per ha (one or less pound per acre) of a compound of this invention would be expected to provide satisfactory weed control, although in some instances application rates in excess of 1.12 Kg per ha (one pound per acre), e.g., up to 5.6 or more Kg per ha (5 or more pounds per acre) might be required. Of course, the efficacy of a particular compound against a particular weed species may readily be determined by routine laboratory or field testing in a manner well known to the art. It is expected that satisfactory weed control can be had at a rate of application in the range of 0.11 to 2.24 Kg per ha (0.1 to 2.0 pounds per acre).

Of course, a compound of this invention can be formulated according to routine methods with any of several known and commonly used herbicidal diluents, adjuvants and carriers. The formulations can contain liquid carriers and adjuvants and carriers. The formulations can contain liquid carriers and adjuvants such as

organic solvents, as well as emulsifiers, stabilizers, dispersants, suspending agents, spreaders, penetrants, wetting agents and the like. Typical carriers utilized in dry formulations include clay, talc, diatomaceous earth, silica and the like. Preferred formulations are those in the form of wettable powders, flowables, dispersible granulates or aqueous emulsifiable concentrates which can be diluted with water at the site of application. Also, dry formulations such as granules, dusts, and the like, may be used.

When desired, a compound of this invention can be applied in combination with other herbicidal agents in an effort to achieve even broader vegetative control. Typical herbicides which can be conveniently combined with Formula I compound include atrazine, hexazinone, metribuzin, ametryn, cyanazine, cyprazine, prometon, prometryn, propazine, simazine, terbutryn, propham, alachlor, acifluorfen, bentazon, metolachlor and N,N-dialkyl thiocarbamates such as EPTC, butylate or vernolate. These, as well as other herbicides described, for example, in the Herbicide Handbook of the Weed Science Society of America, may be used in combination with a compound or compounds of the invention. Typically such formulations will contain from 5 to 95 percent by weight of a compound of this invention.

The herbicidal formulations contemplated herein can be applied by any of several method known to the art. Generally, the formulation will be surfaced applied as an aqueous spray. Such application can be carried out by conventional ground equipment, or if desired, the sprays can be aerially applied. Soil incorporation of such surface applied herbicides is accomplished by natural leaching, and is of course facilitated by natural rainfall and melting snow. If desired, however, the herbicides can be incorporated into the soil by conventional tillage means.

Compounds of this invention are believed effective for preemergence or postemergence control of a wide variety of broadleaf and grassy weeds. Typical of the various species of vegetative growth that may be controlled, combated, or eliminated are, for example, annuals such as pigweed, lambsquarters, foxtail, crabgrass, wild mustard, field pennycress, ryegrass, goose grass, chickweed, wild oats, velvetleaf, purslane, barnyardgrass, smartweed, knotweed, cocklebur, kochia, medic, ragweed, hemp nettle, spurrey, pondweed, carpetweed, morningglory, ducksalad, cheatgrass, fall panicum, jimsonweed, witchgrass, watergrass, wild turnip, and similar annual grasses and weeds. Biennials that may be controlled include wild barley, campion, burdock, bull thistle, roundleaved mallow, purple star thistle, and the like. Also controlled by the compounds of this invention are perennials such as quackgrass, Johnsongrass, Canada thistle, curly dock, field chickweed, dandelion, Russian knapweed aster, horsetail, ironweed, sesbania, cattail, wintercress, horsenettle, nutsedge, milkweed, sicklepod, and the like.

The compounds prepared as described in the Examples were individually screened for herbicidal efficacy, against a variety of broadleaf and grassy weed species, under controlled laboratory conditions of light, humidity and temperature. Solvent solutions of said compounds were applied, both preemergence and postemergence, to test flats containing the various weed species, and herbicidal efficacy was determined by periodic visual inspection, after application of the compounds. Herbicidal efficacy was determined on a Numerical Injury Rating (NIR) scale of from 0 (no injury) to 10 (all plants dead). A NIR rating of 7-9 indicates severe injury; a NIR rating of 4-6 indicates moderate injury, i.e., plant growth is reduced to the extent that normal growth would be expected only under ideal conditions; and a NIR rating of 1-3 indicates slight injury.

For example, the following table gives the average preemergence and/or postemergence NIR determined for each of the compounds prepared as described in Examples I through III on the broadleaf (BL) and grassy (GR) weed species to which the compounds were applied. Each compound was applied at the indicated rate of application in Kg per ha (pounds per acre) and the NIR was determined three weeks subsequent to application.

| | I | II | III |
|---|---|---|---|
| Pre-BL | 9.0 | 6.3 | 8.3 |
| Pre-GR | 9.0 | 9.0 | 8.2 |
| Post-BL | 6.6 | 7.2 | 6.7 |
| Post-GR | 8.2 | 3.0 | 3.3 |
| Rate | 0.56 (0.5) | 2.24 (2) | 2.24 (2) |

The broadleaf weeds used in the screening tests were coffeeweed, jimsonweed, tall morningglory, wild

mustard, teaweed and velvetleaf. The grassy weeds used in the screening tests were barnyardgrass, large crabgrass, Johnsongrass, wild oats and yellow foxtail. In addition to the above observed herbicidal activities the compounds were found very effective, especially when applied preemergence, in controlling yellow nutsedge, a very difficult weed to control.

## Claims

### Claims for the following Contracting States : BE, CH, DE, FR, GB, GR, IT, LI, NL, SE

**1.** A compound represented by the formula:

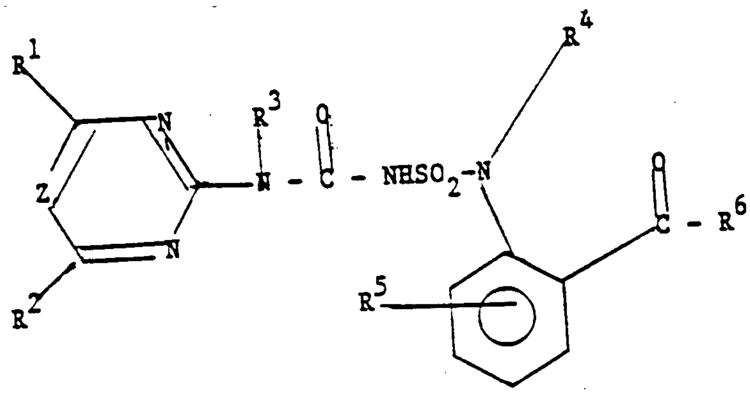

wherein:

| | |
|---|---|
| Z | is N or CH; |
| $R^1$ and $R^2$ | are the same or different and represent halogen or $C_1$ to $C_6$ alkyl or alkoxy; |
| $R^3$ and $R^4$ | are the same or different and represent hydrogen, $C_1$ to $C_4$ alkyl, alkoxyalkyl, haloalkyl, or up to $C_3$ alkenyl or alkynyl; |
| $R^5$ | is hydrogen, halogen or $C_1$ to $C_4$ alkyl, haloalkyl; and |
| $R^6$ | is methyl or ethyl. |

**2.** A compound of Claim 1 wherein Z is nitrogen, $R^1$ is $C_1$ to $C_6$ alkyl, $R^2$ is $C_1$ to $C_6$ alkoxy, $R^3$, $R^4$ and $R^5$ are hydrogen and $R^6$ is methyl.

**3.** A compound of Claim 2 which is 1-[(o-acetylphenyl)-sulfamoyl]-3-(4-methyl-6-methoxy-1,3,5-triazin-2-yl)urea.

**4.** A hericidal formulation containing an agronomically acceptable carrier and a compound or mixture of compounds defined in Claim 1.

**5.** A method of controlling weeds wherein a herbicidally effective amount of herbicide is applied to a growth medium prior to emergence of weeds therefrom or to the weeds subsequent to their emergence from the growth medium,
**characterized by**
using as the herbicide a compound or mixture of compounds defined in Claim 1.

### Claims for the following Contracting State : AT

**1.** A process for preparing sulfamoyl urea derivatives,
**characterized in**
that it leads to a compound represented by the formula I

I

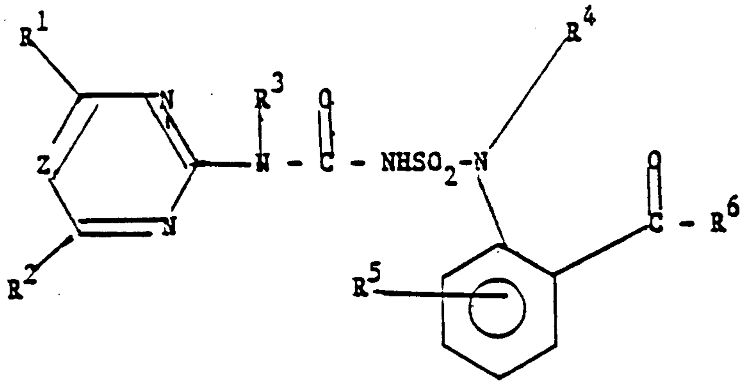

wherein:

| | |
|---|---|
| Z | is N or CH; |
| $R^1$ and $R^2$ | are the same or different and represent halogen or $C_1$ to $C_6$ alkyl or alkoxy; |
| $R^3$ and $R^4$ | are the same or different and represent hydrogen, $C_1$ to $C_4$ alkyl, alkoxyalkyl, haloalkyl, or up to $C_3$ alkenyl or alkynyl; |
| $R^5$ | is hydrogen, halogen or $C_1$ to $C_4$ alkyl, haloalkyl; and |
| $R^6$ | is methyl or ethyl. |

by reacting first an amino pyrimidine or amino triazine of the formula II:

II

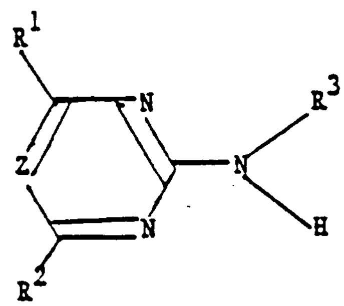

wherein $R^1$, $R^2$, $R^3$ and Z are previously defined, with a halosulfonyl isocyanate of the formula OCN-$SO_2$-Hal, wherein Hal is halogen to form the corresponding halosulfonyl urea of the formula III:

III.

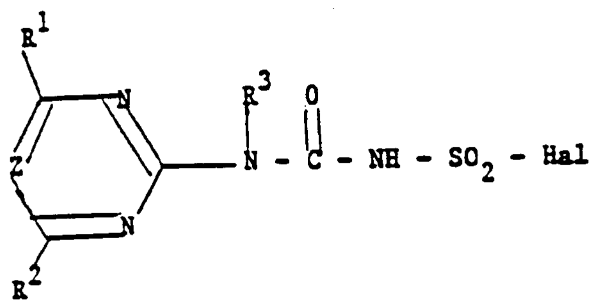

then reacting the formula III compound with at least a stoichiometric amount of a suitably substituted amine of the formula IV:

IV.

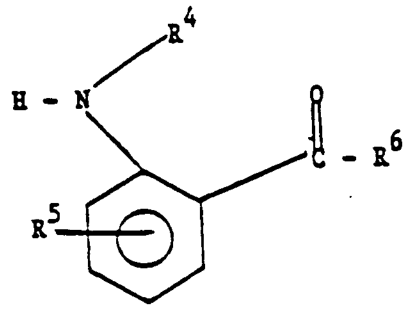

wherein $R^4$, $R^5$ and $R^6$ are as previously defined, to form a formula I compound.

**2.** The process of Claim 1 wherein Z is nitrogen, $R^1$ is $C_1$ to $C_6$ alkyl, $R^2$ is $C_1$ to $C_6$ alkoxy, $R^3$, $R^4$ and $R^5$ are hydrogen and $R^6$ is methyl.

**3.** The process of claim 1 wherein the compounds of the formula III and of the formula IV are reacted in the presence of an acid acceptor.

**4.** The process of claim 2 or 3 which leads to 1-[(o-acetylphenyl)-sulfamoyl]-3-(4-methyl-6-methoxy-1,3,5-triazin-2yl)urea.

**5.** A herbicidal formulation containing an agronomically acceptable carrier and a compound or mixture of compounds prepared as in claim 1.

**6.** A method of controlling weeds wherein a herbicidally effective amount of a herbicide is applied to a growth medium prior to emergence of weeds therefrom or to the weeds subsequent to their emergence from the growth medium,
**characterized by**
using as the herbicide a compound or mixture of compounds prepared as in claim 1.

**Revendications**

**Revendications pour les Etats contractants suivants : BE, CH, DE, FR, GB, GR, IT, LI, NL, SE**

**1.** Un composé représenté par la formule :

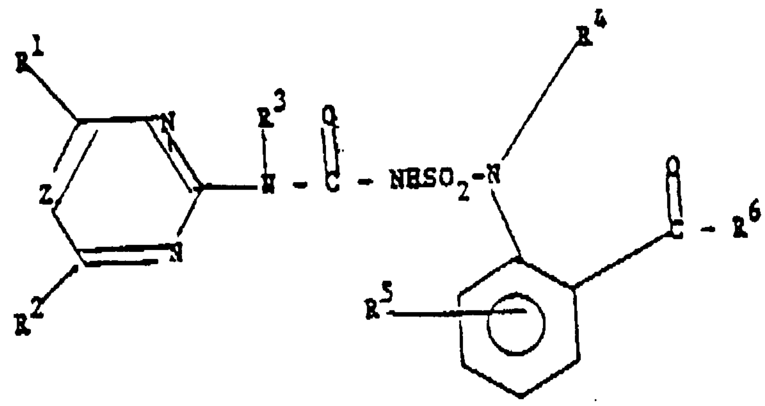

dans laquelle :

| | |
|---|---|
| Z | est N ou CH; |
| $R^1$ et $R^2$ | sont identiques ou différents et représentent un halogène ou un groupe alkyle ou alkoxy ayant de 1 à 6 atomes de carbone; |
| $R^3$ et $R^4$ | sont identiques ou différents et représentent de l'hydrogène, un groupe alkyle en $C_1$ à $C_4$, alkoxyalkyle, haloalkyle, ou un groupe alkényle ou alkynyle ayant jusqu'à 3 atomes |

de carbone ;

$R^5$ est de l'hydrogène,un halogène ou un groupe alkyle en $C_1$ à $C_4$, un groupe haloakyle ; et

$R^6$ est un groupe méthyle ou éthyle.

**2.** Un composé selon la Revendication 1 dans lequel Z est de l'azote, $R^1$ est un groupe alkyle ayant de 1 à 6 atomes de carbone, $R^2$ est un groupe alkoxy ayant de 1 à 6 atomes de carbone, $R^3$, $R^4$ et $R^5$ sont de l'hydrogène et $R^6$ est un groupe méthyle.

**3.** Un composé selon la Revendication 2 qui est la 1-[(o-acétylphényl)-sulfamoyl]-3-(4-méthyl-6-méthoxy-1,3,5-triazin-2-yl)urée.

**4.** Une formulation herbicide contenant un véhicule agronomiquement acceptable et un composé ou mélange de composés définis dans la Revendication 1.

**5.** Une méthode pour lutter contre des adventices selon laquelle on applique une quantité d'herbicide efficace en tant qu'herbicide à un milieu de culture avant l'émergence des adventices de celui-ci ou aux adventices subséquemment à leur émergence du milieu de culture, caractérisée en ce qu'on utilise comme herbicide un composé ou un mélange de composés définis dans la Revendication 1.

**Revendications pour l'Etat contractant suivant : AT**

**1.** Un procédé pour préparer des dérivés de sulfamoyl urée, caractérisé en ce qu'il conduit à un composé représenté par la formule I :

I

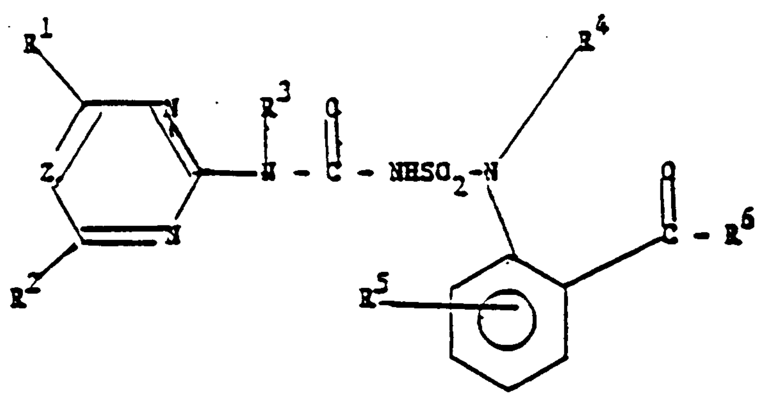

dans laquelle :

Z est N ou CH;

$R^1$ et $R^2$ sont identiques ou différents et représentent un halogène ou un groupe alkyle ou alkoxy ayant de 1 à 6 atomes de carbone;

$R^3$ et $R^4$ sont identiques ou différents et représentent de l'hydrogène, un groupe alkyle en $C_1$ à $C_4$, alkoxyalkyle, haloalkyle, ou un groupe alkényle ou alkynyle ayant jusqu'à 3 atomes de carbone ;

$R^5$ est de l'hydrogène, un halogène ou un groupe alkyle en $C_1$ à $C_4$, un groupe haloakyle ; et

$R^6$ est un groupe méthyle ou éthyle,

en faisant réagir une amino pyrimidine ou une amino triazine de formule II :

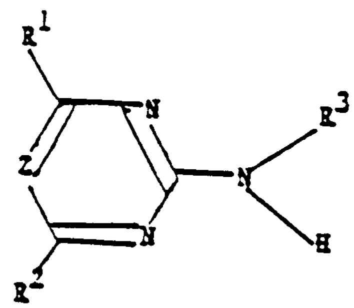

II

dans laquelle $R^1$, $R^2$, $R^3$ et Z sont comme définis précédemment, avec un isocyanate d'halosulfonyle de formule $OCN-SO_2-Hal$, dans laquelle Hal est un halogène, pour former l'halosulfonyle urée correspondante de formule III :

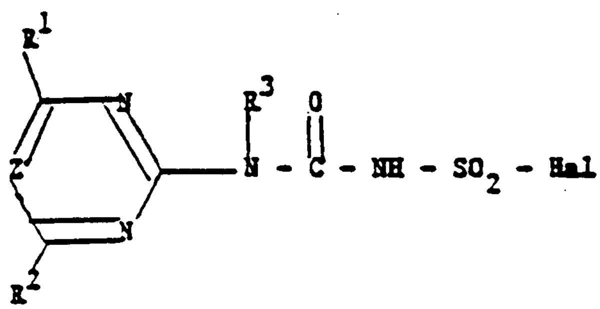

III

puis en faisant réagir le composé de formule III avec au moins une quantité stoechiométrique d'une amine convenablement substituée de formule IV :

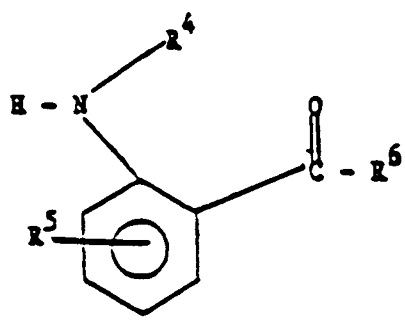

IV

dans laquelle $R^4$, $R^5$ et $R^6$ sont comme définis précédemment, pour former un composé de formule I.

**2.** Le procédé selon la Revendication 1 dans lequel Z est de l'azote, $R^1$ est un groupe alkyle ayant de 1 à 6 atomes de carbone, $R^2$ est un groupe alkoxy ayant de 1 à 6 atomes de carbone, $R^3$, $R^4$ et $R^5$ sont de l'hydrogène et $R^6$ est un groupe méthyle.

**3.** Le procédé selon la Revendication 1 dans lequel on fait réagir les composés de formule III et de formule IV en présence d'un accepteur acide.

**4.** Le procédé selon la Revendication 2 ou 3 qui mène à la 1-[(o-acétylphényl)-sulfamoyl]-3-(4-méthyl-6-méthoxy-1,3,5-triazin-2-yl)urée.

**5.** Une formulation herbicide contenant un véhicule agronomiquement acceptable et un composé ou mélange de composés préparés selon la Revendication 1.

**6.** Une méthode pour lutter contre des adventices selon laquelle on applique une quantité d'un herbicide

efficace en tant qu'herbicide à un milieu de culture avant l'émergence des adventices de celui-ci ou aux adventices subséquemment à leur émergence du milieu de culture, caractérisée en ce qu'on utilise comme herbicide un composé ou un mélange de composés préparés selon la Revendication 1.

**Patentansprüche**
**Patentansprüche für folgende Vertragsstaaten : BE, CH, DE, FR, GB, GR, IT, LI, NL, SE**

1. Verbindung der Formel

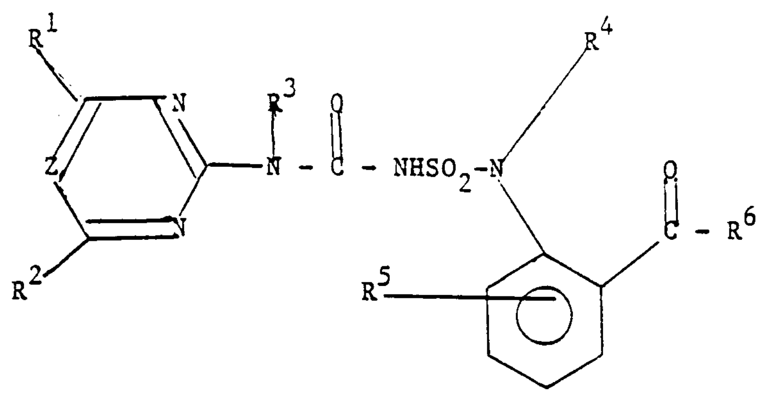

in der Z N oder CH ist,
$R^1$ und $R^2$ gleich oder unterschiedlich sind und Halogen oder $C_1$ bis $C_6$ Alkyl oder Alkoxy sind,
$R^3$ und $R^4$ gleich oder unterschiedlich sind und Wasserstoff, $C_1$ bis $C_4$ Alkyl, Alkoxyalkyl, Haloalkyl oder bis zu $C_3$ Alkenyl oder Alkynyl sind,
$R^5$ ist Wasserstoff, Halogen oder $C_1$ bis $C_4$ Alkyl, Haloalkyl und
$R^6$ ist Methyl oder Ethyl.

2. Verbindung nach Anspruch 1,
**dadurch gekennzeichnet,**
daß Z Stickstoff ist, $R^1$ $C_1$ bis $C_6$ Alkyl ist, $R^2$ $C_1$ bis $C_6$ Alkoxy ist, $R^3$, $R^4$ und $R^5$ Wasserstoff sind und $R^6$ Methyl ist.

3. Verbindung nach Anspruch 2,
**dadurch gekennzeichnet,**
daß sie 1-[(o-Acetylphenyl)-sulfamoyl]-3-(4-methyl-6-methoxy-1,3,5-triazin-2-yl)harnstoff ist.

4. Herbizide Formulierung, enthaltend einen landwirtschaftlich akzeptablen Träger und eine Verbindung oder Mischung von Verbindungen, wie in Anspruch 1 angegeben.

5. Verfahren zum Steuern des Wachstums von Unkräutern durch Aufbringen einer herzbizidwirkenden Menge eines Herbizids auf die Erde vor dem Auflaufen von Unkräutern oder auf die Unkräuter nach deren Auflaufen aus der Erde,
**gekennzeichnet durch**
Verwenden einer Verbindung oder Mischung von Verbindungen, wie in Anspruch 1 angegeben, als Herbizid.

**Patentansprüche für folgenden Vertragsstaat : AT**

1. Verfahren zum Herstellen von Sulfamoylharnstoffderivaten,
**dadurch gekennzeichnet,**
daß es zu einer Verbindung der Formel I führt

I.

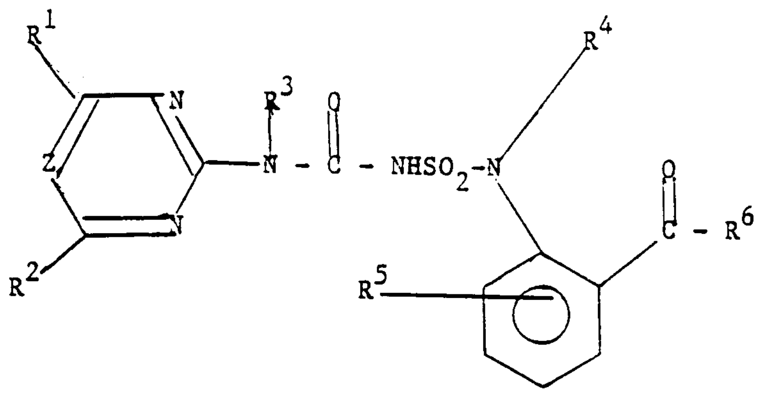

in der Z N oder CH ist,
$R^1$ und $R^2$ gleich oder unterschiedlich sind und Halogen oder $C_1$ bis $C_6$ Alkyl oder Alkoxy sind,
$R^3$ und $R^4$ gleich oder unterschiedlich sind und Wasserstoff, $C_1$ bis $C_4$ Alkyl, Alkoxyalkyl, Haloalkyl oder bis zu $C_3$ Alkenyl oder Alkynyl sind,
$R^5$ ist Wasserstoff, Halogen oder $C_1$ bis $C_4$ Alkyl, Haloalkyl und
$R^6$ ist Methyl oder Ethyl,
durch als erstes Umsetzen eines Aminopyrimidins oder Aminotriazins der Formel II

II.

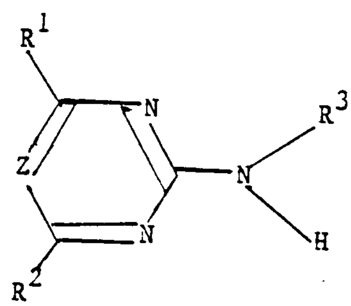

in der $R^1$, $R^2$, $R^3$ und Z die zuvor angegebene Bedeutung haben, mit einem Halosulfonylisocyanat der Formel OCN-$SO_2$-Hal, in der Hal Halogen ist, um den entsprechenden Halosulfonylharnstoff der Formel III zu bilden

III.

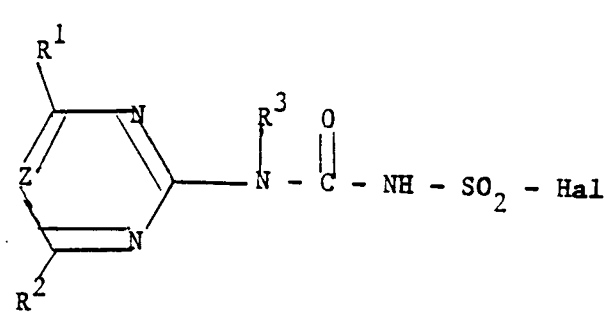

dann Umsetzen der Verbindung der Formel III mit mindestens einer stöchiometrischen Menge eines geeigneten substituierten Amins der Formel IV

IV.

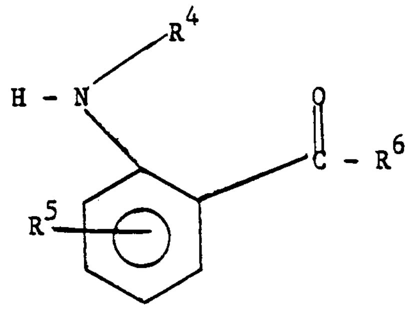

in der $R^4$, $R^5$ und $R^6$ die zuvor angegebene Bedeutung haben, um eine Verbindung der Formel I auszubilden.

**2.** Verfahren nach Anspruch 1,
**dadurch gekennzeichnet,**
daß Z Stickstoff ist, $R^1$ $C_1$ bis $C_6$ Alkyl ist, $R^2$ $C_1$ bis $C_6$ Alkoxy ist, $R^3$, $R^4$ und $R^5$ Wasserstoff sind und $R^6$ Methyl ist.

**3.** Verfahren nach Anspruch 1,
**dadurch gekennzeichnet,**
daß die Verbindungen der Formel III und der Formel IV in Gegenwart eines sauren Akzeptors umgesetzt werden.

**4.** Verfahren nach Anspruch 2 oder 3,
**dadurch gekennzeichnet,**
daß es zu 1-[(o-Acetylphenyl)-sulfamoyl]-3-(4methyl-6-methoxy-1,3,5-triazin-2-yl)harnstoff führt.

**5.** Herbizide Formulierung, enthaltend einen landwirtschaftlich akzeptablen Träger und eine Verbindung oder Mischung von Verbindungen, hergestellt wie in Anspruch 1 angegeben.

**6.** Verfahren zum Steuern des Wachstums von Unkräutern durch Aufbringen einer herzbizidwirkenden Menge eines Herbizids auf die Erde vor dem Auflaufen von Unkräutern oder auf die Unkräuter nach deren Auflaufen aus der Erde,
**gekennzeichnet durch**
Verwenden einer Verbindung oder Mischung von Verbindungen, hergestellt wie in Anspruch 1 angegeben, als Herbizid.